# EUROPÄISCHE PATENTSCHRIFT

(11) **EP 1 318 204 B1**
(45) Veröffentlichungstag und Bekanntmachung des Hinweises auf die Patenterteilung: **24.05.2006**
(21) Anmeldenummer: 02027050.0
(22) Anmeldetag: 03.12.2002
(51) Int. Cl.: C22C 14/00, C22C 16/00

(54) **Titan-Zirkonium-Legierung**
Titanium-Zirconium alloy
Alliage à base de titane et de zirconium

(30) Priorität: 04.12.2001 DE 10159428
(43) Veröffentlichungstag der Anmeldung: 11.06.2003
(73) Patentinhaber: SHERA-Werkstofftechnologie GmbH & Co.KG, 49448 Lemförde (DE)
(72) Erfinder: Nowack, Norbert, Prof. Dr.-Ing., 47877 Willich-Aurath (DE); Grill, Günter, 49448 Lemförde (DE)
(74) Vertreter: Stenger, Watzke & Ring

(56) Entgegenhaltungen:
- EP-A- 1 046 722
- WO-A-97/29624
- GB-A- 1 305 879
- US-A- 2 892 705
- THADDEUS B. MASSALSKI: "Binary Alloy Phase Diagram" Dezember 1990 (1990-12) , ASM INTERNATIONAL , U.S.A. XP002232492 ISBN: 0-87170-406-4 * Seite 3502, Tabelle * * Seite 3503, Zustandsdiagramm *
- PATENT ABSTRACTS OF JAPAN vol. 1995, no. 10, 30. November 1995 (1995-11-30) -& JP 07 188876 A (TAKESHI MASUMOTO;OTHERS: 02), 25. Juli 1995 (1995-07-25)

## Beschreibung

Die vorliegende Erfindung betrifft die Verwendung einer Titan (Ti)-Zirkonium (Zr)-Legierung zum Feingiessen, insbesondere zur Herstellung von Feingußteilen für Dentalzwecke.

In der Medizintechnik und dabei insbesondere in der Dentaltechnik werden Prothetikteile, wenn diese individuell für jeden Patienten hergestellt werden müssen, aus Titan (Ti) oder dessen Legierungen gefertigt, indem entsprechende Gußformen geschaffen werden, in die das flüssige Ti oder dessen Legierungen gegossen werden. Nach dem Erstarren des Titans oder dessen Legierung wird die Gußform entfernt und man erhält das Gußteil. Zum Herstellen dieser Gußformen werden Gußeinbettmassen sowie Gußfeineinbettmassen verwendet, die auf einem Keramiksystem basieren.

Feingußteile aus Ti oder dessen Legierungen konnten insbesondere eine breite Anwendung in Bereichen der Medizintechnik für Implantatteile, Zahnersatz und dergleichen finden, denn Ti und dessen Legierungen zeichnen sich durch eine außerordentlich hohe Korrosionsbeständigkeit im menschlichen Körper und durch ein hohes Maß an Biokompatibilität aus. Diese äußerst positiven Eigenschaften sind verbunden mit einer niedrigen Dichte, die etwa halb so groß ist wie bei den gewöhnlichen Legierungen auf Basis von Eisen (Fe), Nickel (Ni) oder Cobalt (Co), die derzeit in medizinisch-technischen Bereichen eingesetzt werden. Schließlich zeichnen sich Titan und dessen Legierungen durch hohe Festigungswerte aus.

Bislang wurden Titanteile, beispielsweise künstliche Gelenke, Knochenteile und dergleichen, nur durch Schmieden, Schneiden, Fräsen oder Schleifen hergestellt. Die Herstellung von kleinen, dünnwandigen und filigranen Titanteilen, wie sie im medizinischen Bereich und dabei insbesondere in der Dentaltechnik sowie auch im Bereich der Herstellung von Schmuck und Uhrenteilen gefordert werden, ist bislang noch nicht gelungen. Die Gründe hierfür lassen sich auf die schwierige Feingießtechnik für Titan bzw. dessen Legierungen zurückführen. Insbesondere laufen beim Vergießen von Titan oder dessen Legierungen zahlreiche chemische Reaktionen zwischen der keramischen Gußform und dem flüssigen Metall ab, die zu Versprödungen des Titans bzw. dessen Legierungen sowie zu einem Verlust der hohen Korrosionsbeständigkeit und der positiven mechanischen Eigenschaften führen, so daß die Feingußteile unbrauchbar sind. Die Oberfläche des Titangußteils müßte wegen der Versprödung durch Beizen oder Schleifen nachgearbeitet bzw. abgearbeitet werden. Im Bereich der Medizintechnik würden jedoch die Feingußteile dadurch inakzeptabel in ihren Geometrien verändert. Bei dünnen Teilen, wie sie beispielsweise im Bereich der Dentaltechnik vorkommen und wo die Wandstärke nur einige Millimeter beträgt, könnte die abzuarbeitende Schicht die Stärke des Gußteils besitzen, was alle dentaltechnischen Arbeiten ausschließen würde. Eine funktionierende Gießtechnik von dünnen, meist kleinen und filigranen hochpräzisen Teilen ist somit die Voraussetzung für eine Anwendung im medizinischen Bereich, zumal die Gußteile individuell angefertigt werden müssen, da sie dem jeweiligen Patienten im Körper angepaßt werden müssen. Dies setzt weiterhin eine hohe Gußpräzision voraus.

GB 1 305 879 betrifft eine binäure. Titan - Zirkonium-Legierung enthaltend Zr 25-75 Gew.-%. Rest Ti und weniger als 3 Gew.% übliche Verunreinigungen. Diese Legierung wird für Gussteile für Dentalimplantate oder Dentalprothesen verwendet.

Die beschriebenen Probleme bei Titan-Feingußteilen liegen, wie bereits erwähnt, in der Entstehung einer versprödeten Oberfläche, die in der Technik "alpha-case" genannt wird. Damit ist die Bildung einer Randzone auf dem Titangußstück gemeint, in der dieses durch eine Sauerstoffaufnahme infolge einer chemischen Verschlackungreaktion stark versprödet ist, wobei die Schichtdicke je nach Gußteilgeometrie, Gießbedingungen und Verfahrenstechnik Stärken von 0,1 mm bis 2,0 mm besitzt. Diese alpha-case-Schicht entsteht durch chemische Reaktionen zwischen dem flüssigen Titan bzw. der flüssigen Titanlegierung und der keramischen Formmasse beim Feingießen des Gußteils. Hiervon sind insbesondere feine, kleine und filigrane Teile betroffen.

Beim Gießen kommt üblicherweise Titan in den Reinheitsstufen von 99,2% bis 99,5% zur Anwendung, welches eine Liquidustemperatur von etwa 1680°C aufweist. Um das Titan bzw. dessen Legierungen beim Gießprozeß einwandfrei in die Gußform überführen zu können, muß beim Gießprozeß die Gießtemperatur etwa 50°C über der Liquidustemperatur, also bei etwa 1730°C liegen. Derartig hohe Temperaturen belasten sowohl den Schmelzapparat, meist ein unter Reinst-Argon (99,9995%) betriebener Lichtbogenofen, als auch die Gußform selbst. Bei Gießtemperaturen von 1650°C bis 1800°C nehmen das Titan bzw. dessen Legierungen infolge der hohen Sauerstoffaffinität des Titans Sauerstoff (O) sowie die entsprechende Metallkomponente, die das Oxid in der Keramikmasse bildet, auf. Dieser Vorgang findet auch dann statt, wenn unter einer Inertgasatmosphäre, beispielsweise unter Reinst-Argon, gegossen wird. Der Sauerstoff (O) verursacht dabei die hohe Versprödung, da schon geringe Gehalte von interstitiell eingelagerten Sauerstoffatomen im Metallgitter des Titans (Ti) eine starke Abnahme der Duktilität zur Folge haben. Anzustreben ist deshalb eine möglichst kurze Aufschmelzphase für das Titan bzw. dessen Legierungen, um damit die Aufnahme von Sauerstoff aus der Gasphase zu verringern.

Der Erfindung liegt in Anbetracht dieses Standes der Technik die **Aufgabe** zugrunde, eine Titan-Legierung zur Verwendung zur Herstellung von Feingußteilen für Dentalzwecke, zu schaffen, welche gegenüber reinem Titan eine wesentlich niedrigere Liquidustemperatur und eine mit reinem Titan vergleichbar hohe Biokompatibilität und Korrosionsbeständigkeit aufweist.

Die Aufgabe wird durch die Verwendung einer Titan-Zirkonium-Legierung zum Feingiessen **gelöst**, die gekennzeichnet ist durch einen Gehalt an Titan (Ti) im Bereich zwischen maximal 70 Gew.-% und minimal 35 Gew.-%, Rest Zirkonium (Zr) und übliche Verunreinigungen.

In einer vorteilhaften Ausgestaltung der Erfindung sind Gehalte an Titan (Ti) und/oder Zirkonium (Zr) im Bereich von 0,01 Gew.-% bis 10 Gew.-% ersetzt durch Zusätze mit Zinn (Sn) und/oder Aluminium (Al) und/oder Silber (Ag) und/oder Gold (Au) und/oder Palladium (Pd) einzeln oder in Kombination oder gemeinsam.

Der Erfindung liegt die Erkenntnis zugrunde, daß durch wesentlich niedrigere Liquidustemperaturen eine erniedrigte Feingießtemperatur erzielbar ist, die zu einer drastischen Verringerung der Sauerstoffaufnahme während des Gießvorganges und damit zu einer verringerten Versprödung führt.

Um die hohe Biokompatibilität und Korrosionsbeständigkeit im Vergleich zu Titan (Ti) aufrechtzuerhalten, weist die erfindungsgemäße Ti-Zr-Legierung als Zusatzstoffe vorteilhafterweise Zinn, Aluminium, Silber, Gold und/oder Palladium auf. Diese sind als Legierungsadditive biokompatibel und weisen eine hohe Löslichkeit im Legierungssystem Titan-Zirkonium auf. Die Edelmetalle Rhodium (Rh), Platin (Pt) und Iridium (Ir) wären als Legierungsadditive zwar ebenfalls biokompatibel, besitzen jedoch entweder nur geringe Löslichkeiten im Legierungssystem Titan-Zirkonium, d.h. sie neigen zur Bildung von intermetallischen Phasen mit der Folge der Versprödung, oder der erfindungsgemäße temperaturerniedrigende Effekt hinsichtlich der Erstarrungstemperatur der Legierung ist zu gering. Für eine effektive Verringerung und damit Beseitigung des durch Sauerstoffaufnahme während des Feingießprozesses verursachten alpha-case-Bildung ist entscheidend, daß die zu vergießende Titan-Legierung eine möglichst niedrig Feingießtemperatur besitzt. Vorteilhafterweise liegt die Erstarrungstemperatur der Legierung in einem Bereich zwischen 1400°C und 1580°C.

Es wurde festgestellt, daß das reine Zweistoffsystem Titan-Zirkonium hierfür die ideale Ausgangsbasis bietet. Im Konzentrationsbereich um 51 Gew.-% Zirkonium in Titan durchläuft dieses Zweistoffsystem überraschender Weise ein breites Liquidusminimum bei etwa 1560°C. Die Erstarrungstemperatur der Titan-Zirkonium-Legierung ist in diesem Bereich gegenüber der Erstarrungstemperatur reinen Titans um etwa 120°C erniedrigt. Titan und Zirkonium bilden ein völlig isomorphes System, welches im Konzentrationsbereich um 51 Gew.-% Zirkonium in Titan bei 1560°C ohne Seigerungen erstarrt. Es wurde festgestellt, daß das Erstarrungsintervall in diesem Konzentrationsbereich null ist. Eine Entmischungstendenz zwischen Titan und Zirkonium ist im gesamten Phasensystem nicht vorhanden. Dies ist eine ideale Grundlage für eine Feingußlegierung. Die erfindungsgemäßen Titan-Zirkonium-Legierungen sind einphasig. Ihre Kristallstruktur im Hochtemperaturbereich ist kubisch-raumzentriert mit einem β-(Ti, Zr)-Substitutionsmischkristall. Im Bereich um 600°C wandelt sich der β-(Ti, Zr)-Substitutionsmischkristall isomorph in den alpha-(Ti, Zr)-Substitutionsmischkristall um, der eine hexagonal-dichte Packung besitzt. Die Legierungen bleiben in jedem Falle einphasig. Bei der β/α-Umwandlung bleiben bei einer entsprechenden Abkühlungsgeschwindigkeit kleine Anteile der gelösten biokompatiblen Legierungsadditive Zinn, Aluminium, Silber, Gold und/oder Palladium (Zusatzstoffe) gelöst, so daß die Legierung in jedem Temperaturbereich einphasig ist. Bei Übersättigung mit den Zusatzstoffen Zinn, Aluminium, Silber, Gold und/oder Palladium existieren auch metastabile Zustände, so daß bei der α-Umwandlung während der Abkühlungsphase eines Gußteiles diese Legierungsadditive, ähnlich wie bei einer martensitischen Umwandlung, zwangsgelöst bleiben. Die erfindungsgemäße Legierung bleibt somit auch bei einer Übersättigung mit den Zusatzstoffen Zinn, Aluminium, Silber, Gold und/oder Palladium einphasig.

Weitere Einzelheiten, Merkmale und Vorteile der Erfindung werden nachfolgend anhand der Figuren näher erläutert. Dabei zeigen:
- Fig. 1:: Das Zustandsdiagramm einer Titan-Zirkonium-Legierung mit 0,4 Gew.-% Sauerstoff (O) als Begleitstoff;
- Fig. 2:: das Zustandsdiagramm einer mit Zinn (Sn) versetzten Titan-Zirkonium-Legierung und
- Fig. 3:: ein weiteres Zustandsdiagramm einer mit Zinn (Sn) versetzten Titan-Zirkonium-Legierung.

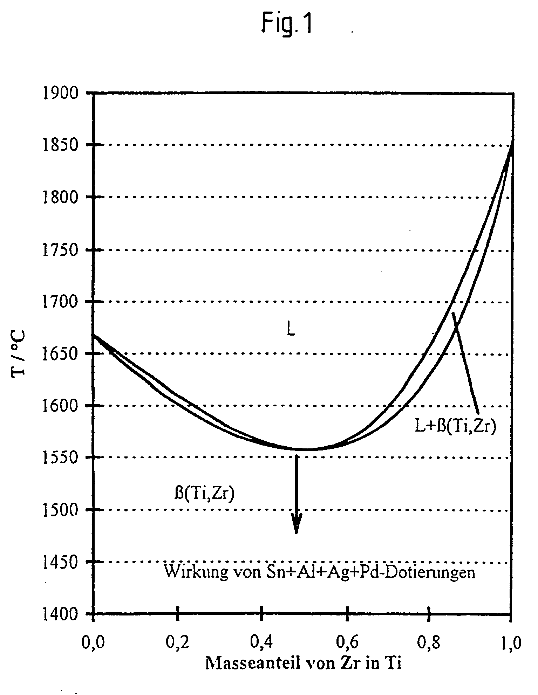

Das Zustandsdiagramm gemäß Fig. 1 zeigt das Erstarrungsverhalten einer Titan-Zirkonium-Legierung mit 0,4 Gew.-% Sauerstoff (O) als übliche Verunreinigung. Anhand von Fig. 1 ist die Einphasigkeit der Titan-Zirkonium-Legierung in jedem Konzentrationsbereich vom Liquidusbereich bis in jeden tieferen Temperaturbereich zu erkennen, welche für die mit der Biokompatibilität gekoppelten hohen Korrosionsbeständigkeit mit entscheidend ist. Zirkonium ist dem Titan in werkstoffkundlicher und chemischer Hinsicht sehr ähnlich. Hinsichtlich der Korrosionsbeständigkeit übertrifft Zirkonium Titan bei weitem, da Zirkonium wie auch Titan eine sehr hohe Passivität aufweist. Nach Tantal (Ta) ist Zirkonium (Zr) das passivste Metall. Die Biokompatibilität wird primär von der Passivität einer Legierung bestimmt. Eine gute Gewebeverträglichkeit ist dann vorhanden, wenn elektrisch schlechtleitende Deckschichten, also passive Schichten, vorliegen. Hierdurch werden Redox-Reaktionen an der Phasengrenze Gewebe/Werkstoff unterbunden. Zirkonium verursacht die Passivität eines Werkstoffes, so daß Zirkonium enthaltende Werkstoffe besonders passiv sind, wie umfassende klinische Erfahrungen bestätigen.

Oxidische Zr(IV)-Verbindungen, die in der Passivschicht enthalten sind, besitzen außerordentlich niedrige Toxizitätseigenschaften, analog den oxidischen Ti(IV)-Verbindungen. Oxidische Zr(IV)- und Ti(IV)-Verbindungen gehören zu den schwerlöslichsten Verbindungen dieser Art, so daß sie vom menschlichen Körper praktisch nicht resorbiert werden. Dies zeichnet die hohe Biokompatibilität von Titan (Ti) und Zirkonium (Zr) und die darauf ableitbaren einphasigen Legierungen aus. Durch die beiden Faktoren "Einphasigkeit" und "Substitution von Titan durch Zirkonium" ist eine hohe Steigerung der Korrosionsbeständigkeit und damit der Biokompatibilität gegeben. Die Anwendung derartiger Gußlegierungen zum Feingielssen von Feingußteilen für medizinische Zwecke, insbesondere Feingußteilen für Dentalzwecke ist bislang nicht bekannt.

Um die Erstarrungstemperatur von Titan-Zirkonium-Legierungen weiter zu erniedrigen, werden diese Legierungen mit den Metallkomponenten Zinn (Sn), Aluminium (Al), Silber (Ag), Gold (Au) und/oder Palladium (Pd) als Legierungsadditiven versehen. Der in Fig. 1 eingezeichnete Pfeil zeigt die Wirkung derartiger Zusaztstoffe mit den Legierungsadditiven hinsichtlich einer Erniedrigung der Erstarrungstemperatur von Titan-Zirkonium-Legierungen mit etwa 50 Gew.-% Titan und 50 Gew.-% Zirkonium.

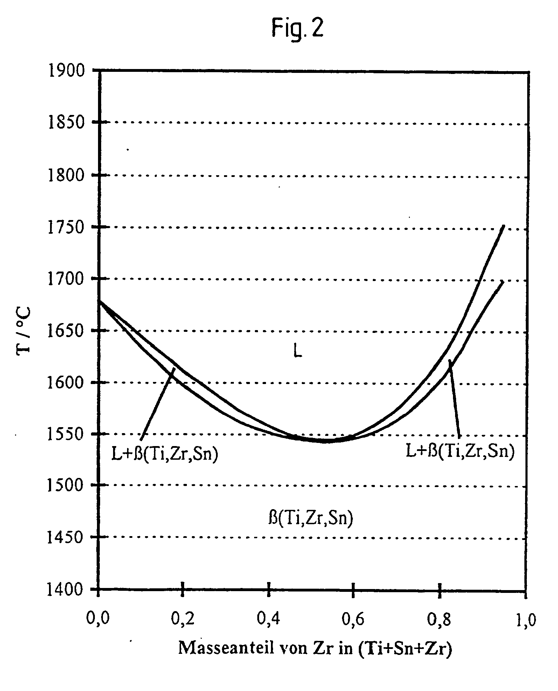

Das Zustandsdiagramm gemäß Fig. 2 zeigt das Erstarrungsverhalten einer Titan-Zirkonium-Legierung, welche mit 5 Gew.-% Zinn (Sn) versehen wurde. Wie anhand von Fig. 2 zu erkennen ist, bleiben durch die Zugabe von Zinn (Sn) das Erstarrungsminimum und die Einphasigkeit der Legierung erhalten. Die Seigerungstendenz bei 54 Gew.-% Zirkonium ist gleich null. Das Erstarrungsminimum wird gegenüber einer Legierung mit 51 Gew.-% Zirkonium (Zr) und 49 Gew.-% Titan (Ti) um etwa 20°C abgesenkt, so daß die Erstarrung bei etwa 1540°C erfolgt. Das Erstarrungsintervall ist praktisch gleich null.

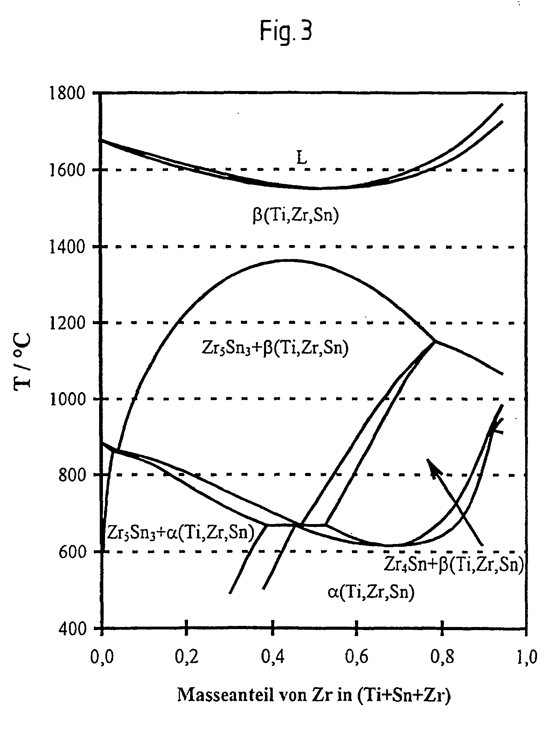

Im Zustandsdiagramm gemäß Fig. 3 sind alle einphasigen und zweiphasigen Bereiche des mit 5 Gew.-% Zinn (Sn) versehenen Legierungssystems Titan-Zirkonium dargestellt. Die dreiphasigen Bereiche ergeben sich aus den Übergangsphasenbereichen.

Wie bereits im Zusammenhang mit Fig. 2 erläutert, erstarrt die Legierung zunächst im Konzentrationsbereich von etwa 50 Gew.-% Zirkonium (Zr). Ausgehend vom Liquidusbereich (L) in den einphasigen β-Substitutionsmischkristallen β (Ti, Zr, Sn) mit der kubisch-raumzentrierten Struktur (bcc-A2-Struktur). Die Erstarrung erfolgt praktisch seigerungsfrei und unter großer Erniedrigung der Schmelzpunkte, verglichen mit den Komponenten Titan und/oder Zirkonium. Das Schmelzintervall ist praktisch null, also ideal für den Verwendungszweck als Feingußlegierung. Bei Abkühlung der festen Legierung scheidet sich vorübergehend ein geringer Anteil intermetallischer Phasen aus der β-Phase aus. In einer titanreicheren Legierung ist dies Zr₅Sn₃ in einer zirkoniumreicheren Legierung ist dies Zr₄Sn. Unterhalb von 600°C wird die Legierung aber wieder einphasig, wie anhand von Fig. 3 zu erkennen. Es entsteht der α-Substitutionsmischkristall alpha (Ti, Zr, Sn) mit der hexagonal-dichten Packung (hcp-A3-Struktur). Derartige aus dem einphasigen α (Ti, Zr, Sn)-Substitutionsmischkristallen bestehenden Legierungen sind äußerst korrosionsstabil und damit biokompatibel und besitzen darüber hinaus keinerlei Versprödungsneigungen, d.h. ein hohes Maß an plastischer Verformbarkeit ist gegeben.

Die Ergebnisse von Zusätzen einer Titan-Zirkonium-Legierung mit den Legierungsadditiven Zinn (Sn), Aluminium (Al), Silber (Ag), Gold (Au) und/oder Palladium (Pd) sind in der nachfolgenden Tabelle wiedergegeben.

| **Zusatz von** | **bei 1400°C [β-(Ti, Zr)-Bereich]** | **bei 500°C [α-(Ti, Zr)-Bereich]** | **Erstarrungs-temperatur** |
|---|---|---|---|
| **Sn** | 13% | 6% | 1530°C / 13% Sn |
| **Ag** | 15% | 3% | 1520°C / 15% Ag |
| **Pd** | 8% | 0,8% | 1530°C / 8% Pd |
| **Al** | 20% | 4% | 1450°C / 20% Al |
| **Au** | 10% | 5% | 1500°C / 10% Au |

Die Tabelle zeigt maximale Löslichkeiten (Gleichgewichte) der Zusätze in einer einphasigen Legierung von 50 Gew.-% Titan und 50 Gew.-% Zirkonium. Verwendet wurde handelsübliches technisches Zirkonium, welches bis zu 2,5 Gew.-% Hafnium (Hf) enthält. Da Hafnium (Hf) und Zirkonium (Zr) sich chemisch und werkstoffkundlich sehr ähnlich sind, ist eine Trennung von Zirkonium (Zr) und Hafnium (Hf) technisch nicht notwendig. Legierungen des Zirkonium enthalten deshalb immer Hafnium (Hf) als vernachlässigbaren Bestandteil.

Unterhalb von 500°C laufen Phasenänderungen praktisch nicht mehr ab. Obwohl eine 50 Gew.-% Titan und 50 Gew.-% Zirkonium enthaltenden Legierung bei 500°C nach thermodynamischen Gesichtspunkten übersättigt sein kann, kommt es in bestimmten Fällen nicht zur Ausscheidung intermetallischer Phasen, sondern der mit Zusatzstoffen versetzte Bestandteil kann im α-(Ti, Zr)-Gitter in einem metastabilen Zustand mit erhöhten Abkühlungsgeschwindigkeiten zwangsgelöst bleiben.

Der kombinierte Einsatz der als Zusätze eingesetzten Metalle Zinn, Aluminium, Silber, Gold und Palladium in einer Basislegierung von 49 Gew.-% Titan und 51 Gew.-% Zirkonium bringt eine starke Absenkung der Erstarrungstemperatur unter Beibehaltung der Einphasigkeit der Legierung mit sich, wie das nachfolgende Beispiel zeigt.

### Beispiel:

Eine Legierung mit 49 Gew.-% Titan und 51 Gew.-% Zirkonium, welche mit 3 Gew.-% Zinn versetzt wurde, wies folgende Eigenschaften auf:

### Feingießverhalten

Bei der Herstellung von Dentalteilen wie Kronen und Brücken durch Feingiessen Erstarrungstemperatur etwa 1550°C (Vergleich: reines Titan etwa 1680°C)

Feingießtemperatur etwa 1600°C (Vergleich: reines Titan etwa 1740°C)

Aufschmelzzeit für ein Feingußteil von etwa 20 g in einem Lichtbogenofen vom Typ MORITA unter einer Argon (Ar)-Atmosphäre und Teilvakuum etwa 40 s (Vergleich: reines Titan etwa 60 s)

Die Legierung ließ sich problemlos ohne alpha-case-Fehler zu dentalen Kronen- und Brückenimplantaten zu jeweils 20 g - Ansätzen vergießen. Die Vorwärmtemperatur der quarzfreien Keramikgußform betrug etwa 650°C.

### Elektrochemische Korrosionsdaten (Biokompatibilität)

In physiologischer NaCI-Lösung bei 37°C unter einer N₂-Atmosphäre.

### Ruhepotential etwa - 50 mV_{H} (Vergleich: reines Titan etwa -100 mV_{H})

Potentiostatisches Durchbruchspotential etwa + 1900 mV_{H} (Vergleich: reines Titan etwa + 2000 mV_{H})

Stromdichte im Passivbereich bei + 800 m V_{H} etwa 10⁻⁷ A/cm² (Vergleich: reines Titan etwa 4x10⁻⁷ A/cm²)

Die in den Figuren dargestellten und beschriebenen Ausführungsbeispiele dienen lediglich der Erläuterung der Erfindung und sind für diese nicht beschränkend.

## Patentansprüche

1. Verwendung einer Titan-Zirkonium-Legierung zum Feingiessen, vorzugsweise für Dentalzwecke, wobei die Legierung einen Gehalt an Titan (Ti) im Bereich zwischen maximal 70 Gew.-% und minimal 35 Gew.-%, Rest Zirkonium (Zr) und übliche Verunreinigungen aufweist, wobei 0,01 Gew.-% bis 10 Gew.-% der Gehalte an Ti und/oder Zr durch Zusätze mit Zinn (Sn) und/oder Aluminium (Al) und/oder Silber (Ag) und/oder Gold (Au) und/oder Palladium (Pd) einzeln oder in Kombination ersetzbar sind.

2. Titan-Zirkonium-Legierung nach Anspruch 1, **dadurch gekennzeichnet, dass** Gehalte an Titan (Ti) und/oder Zirkonium (Zr) im Bereich von 0,01 Gew.-% bis 10 Gew.-% ersetzt sind durch Zusätze mit Zinn (Sn) und/oder Aluminium (Al) und/oder Silber (Ag) und/oder Gold (Au) und/oder Palladium (Pd) einzeln oder in Kombination oder gemeinsam.

3. Titan-Zirkonium-Legierung nach Anspruch 1 oder Anspruch 2, **dadurch gekennzeichnet, daß** dass die Erstarrungstemperatur der Legierung in einem Bereich zwischen 1400°C und 1580°C liegt.

## Claims

1. Use of a titanium-zirconium alloy for investment casting, preferably for dental purposes, wherein the alloy has a titanium (Ti) content in the range between a maximum of 70% by weight and a minimum of 35% by weight, the rest being zirconium (Zr) and usual impurities, wherein 0.01% by weight to 10% by weight of the contents of Ti and/or Zr can be replaced by additions of tin (Sn) and/or aluminium (al) and/or silver (Ag) and/or gold (Au) and/or palladium (Pd) singly or in combination.

2. Titanium-zirconium alloy as claimed in Claim 1, **characterised in that** contents of titanium (Ti) and/or zirconium (Zr) in the range from 0.01% by weight to 10% by weight are replaced by additions of tin (Sn) and/or aluminium (al) and/or silver (Ag) and/or gold (Au) and/or palladium (Pd) singly or in combination or jointly.

3. Titanium-zirconium alloy as claimed in Claim 1 or Claim 2, **characterised in that** the solidification temperature of the alloy lies in a range between 1400 °C and 1580 °C.

## Revendications

1. Utilisation d'un alliage de titane et de zirconium pour des coulées de précision, de préférence pour des usages en dentisterie, lequel alliage a une teneur en titane (Ti) comprise entre 70 % en poids au maximum et 35 % en poids au minimum, le reste étant composé de zirconium (Zr) et des impuretés habituelles, de 0,01 % à 10 % en poids de la teneur en titane et/ou en zirconium pouvant être remplacé par des ajouts d'étain (Sn) et/ou d'aluminium (Al) et/ou d'argent (Ag) et/ou d'or (Au) et/ou de palladium (Pd), isolément ou en combinaison.

2. Alliage de titane et de zirconium selon la revendication 1, **caractérisé en ce que** des teneurs en titane (Ti) et/ou en zirconium (Zr) comprises entre 0,01 % et 10 % en poids sont remplacées par des ajouts d'étain (Sn) et/ou d'aluminium (Al) et/ou d'argent (Ag) et/ou d'or (Au) et/ou de palladium (Pd), isolément ou en combinaison.

3. Alliage de titane et de zirconium selon la revendication 1 ou 2, **caractérisé en ce que** la température de solidification de l'alliage est comprise entre 1400°C et 1580°C.
